# EUROPEAN PATENT APPLICATION

(11) **EP 3 257 538 A1**
(43) Date of publication of application: **20.12.2017**
(21) Application number: 16749296.6
(22) Date of filing: 10.02.2016
(51) Int. Cl.: A61M 5/30

(54) **NEEDLE-FREE INJECTING DEVICE**

(30) Priority: 12.02.2015 JP 2015025687
(71) Applicant: Daicel Corporation, Osaka-shi, Osaka 530-0011 (JP)
(72) Inventor: MASUMOTO, Takaya, Tatsuno-shi Hyogo 671-1681 (JP)
(74) Representative: Jones, Nicholas Andrew
(86) International application number: PCT/JP2016/054025
(87) International publication number: WO 2016/129653

(57) **Abstract**

A needleless injection apparatus is provided which forms a suitable contact state between an injection opening from which an injection objective substance is injected and an injection object. The needleless injection apparatus is provided with an expansible bag body arranged so as to surround an outer peripheral surface of the injection object, a needleless syringe including no injection needle, and a gas supply unit for supplying the expansion gas to the bag body. Here, the needleless syringe has a nozzle portion for injecting the injection objective substance toward the injection object surrounded by the bag body, with its injection opening being in a state opposed to the injection object. Then, the bag body includes a first bag portion arranged at any of locations except an arrangement location of the needleless syringe so as to pressurize the injection object by expansion, and a second bag portion arranged at a location at which the nozzle portion is sandwiched between the second bag portion and the injection object so as to push the injection opening to an injection object side by expansion.

## Description

### TECHNICAL FIELD

The present invention relates to a needleless injection apparatus provided with a needleless syringe including no injection needle.

### BACKGROUND ART

From a hygienic aspect, needleless syringes each including no injection needle are considered as promising, and are widely developed. In such needleless syringes, a pressurized injection solution is injected so as to penetrate through an epidermis of an injection target or object by its injection energy, and components of the injection solution will be delivered into the interior of the injection object. On the other hand, it becomes difficult to deliver the components of the injection solution due to the non-existence of an injection needle, if an injection opening of the needleless syringe is not made into a state of being in suitable contact with the injection object after positioning the needleless syringe with respect to the injection object in an accurate manner.

Here, in patent literature 1, there is disclosed an auxiliary instrument which serves to hold an outlet side end of a needleless syringe so that injection can be made to a user of the needleless syringe at an accurate position. In addition, in patent literature 2, there is disclosed a medical apparatus which has a needleless syringe incorporated therein, together with a blood pressure meter. This medical apparatus senses a blood pressure by means of a sensor so that a patient is automatically administered with a medical fluid from the needleless syringe, wherein the apparatus is fitted on the body of the user, and injection is carried out while measuring the blood pressure, so that the blood pressure can become a suitable blood pressure value.

### PRIOR ART LITERATURES

### PATENT LITERATURES

Patent Literature 1: Japanese translation of PCT international application publication No. 2000-508928
Patent Literature 2: United States Patent No. 8,702,614

### SUMMARY OF THE INVENTION

### PROBLEMS TO BE SOLVED BY THE INVENTION

Although the auxiliary instrument in the above-mentioned patent literature 1 serves to mount the needleless syringe on the injection object, it is basically to carry out the mounting of the needleless syringe in such a manner that the injection opening of the needleless syringe can be positioned at a desired location. However, it can not be confirmed that an injection opening of a nozzle from which the injection solution of the needleless syringe is injected is in abutment with the injection object at a suitable pressure. Accordingly, with this auxiliary instrument alone, it is difficult to realize suitable injection by the needleless syringe.

In addition, with the medical apparatus according to the above-mentioned patent literature 2, there is disclosed a construction in which the injection object of the user is pressurized in order to measure the blood pressure of the user, but no mention is made at all about the pressurization with respect to the needleless syringe, and likewise, even with this medical apparatus, it can not be confirmed whether the contact of a tip end portion of the needleless syringe with the skin of the user is sufficient. Accordingly, in this medical apparatus, too, it is difficult to realize suitable injection by the needleless syringe.

Accordingly, in consideration of the above-mentioned problems, the present invention has for its object to provide a needleless injection apparatus in which a suitable state of contact is formed between an injection opening from which an injection objective substance is injected and an injection object, in order to achieve suitable injection by a needleless syringe.

### MEANS FOR SOLVING THE PROBLEMS

In order to solve the aforementioned problems, the inventor of the subject application adopts a construction in which a bag body is provided for pushing out an injection object to an injection opening of a nozzle portion which injects an injection objective substance, and at the same time for pushing the injection opening to an injection object side, too. Specifically, the present invention resides in a needleless injection apparatus which comprises: an expansible bag body that is adapted to be arranged so as to surround an outer peripheral surface of an injection object; a needleless syringe including no injection needle that comprises a nozzle portion for injecting an injection objective substance in said needleless syringe toward said injection object surrounded by said bag body, said nozzle portion including an injection opening which is in a state opposed to said injection object when said bag body is mounted on said injection object; and a gas supply unit that supplies an expansion gas to said bag body. Then, said bag body includes: a first bag portion that is a bag portion adapted to expand by said expansion gas from said gas supply unit, said first bag portion being arranged at any of locations in said bag body except an arrangement location of said needleless syringe, in such a manner as to pressurize said injection object by expansion thereof; and a second bag portion that is a bag portion adapted to expand by said expansion gas from said gas supply unit, and is formed separately from said first bag portion, said second bag portion being arranged at a location at which said nozzle portion is sandwiched between said second bag portion and said injection object, in such a manner as to push said injection opening toward said injection object by expansion thereof.

With respect to the needleless syringe with which the needleless injection apparatus according to the present invention is provided, a specific structure thereof is not limited in particular as long as it is constructed such that the needleless syringe has the nozzle portion from which the injection objective substance is injected to the injection object, and the injection opening of the nozzle portion is in opposition to the injection object surrounded by the bag body, in a state where the needleless syringe is mounted on the bag body, as mentioned above. For example, as an energy source for injection of the injection objective substance, there can be adopted a variety of kinds of known energy generation modes. As such examples, there can be adopted an ignition charge which is ignited by an igniter, or a gas generating agent which generates gas by combustion. In addition, as other forms of driving portion than the above-mentioned, there may be utilized release of the energy of a resilient member such as a spring, or the energy of a compressed gas, as the energy applied to a holding portion. Alternatively, there may be utilized a magnetic valve, a solenoid actuator, etc., which are driven by application of voltage from a power supply circuit, or there may also be utilized the resilience of an urging spring accumulated, by releasing a piston fixed with the urging spring from its locking state by means of any of these driving sources. Such injection energy will be directly or indirectly transmitted to the injection objective substance, and the injection objective substance thus pressurized will be injected from the injection opening of the nozzle portion toward the injection object.

In addition, with respect to the injection objective substance to be injected from the needleless syringe, it may contain a component which is expected to be effective in a target location of the injection object. Therefore, an accommodation state of the injection objective substance in the syringe and a specific physical form of the injection objective substance such fluid in a liquid or gel form, powder, granular solid is not particularly limited as long as that the injection objective substance can be injected at least by the above mentioned injection construction. For example, the injection objective substance is a liquid, and may be a solid if it is a glue-like roll body or powder which can be injected. The injection objective substance may contain a component which is to be delivered to the target location of the injection object. The component may exist in a state of being dissolved in the injection objective substance or may exist in a simply mixed state without being dissolved. Examples of the component to be delivered include vaccines for enhancing antibody, proteins for cosmetic treatments, and cultured cells for regenerating hair. These components are contained in fluid in a liquid or gel form so that the components can be injected, whereby the injection objective substance is formed. In addition, the injection objective substance may not be a predetermined substance itself, but may be a contained or packed substance such as a capsule containing the predetermined substance, etc. Further, the injection objective substance may not be a medicine which exerts a medical effect in the injection object, but may be a substance which is embedded in a living body for a predetermined purpose. For example, a minute IC chip including a vital identification number, etc., recorded therein or the like may also be used as the injection objective substance.

Here, with the needleless injection apparatus according to the present invention, the needleless syringe is supplied for use in a state of being mounted on the bag body. That is, the needleless syringe does not inject the injection objective substance independently, but an environment for injection of the injection objective substance by the needleless syringe (hereinafter, referred to as an "injection support environment") is formed in a state where the needleless syringe is supported by the first bag portion and the second bag portion with which the bag body is provided, thereby making it possible to achieve a suitable injection result.

Specifically, the first bag portion and the second bag portion are of bag-shaped construction in which they can be expanded or inflated by air supplied from the gas supply unit, and they are formed separately from each other. However, the supply of air from the gas supply unit to the individual bag portions may be carried out with independent paths, and some parts of these supply paths may be connected with each other. Then, the first bag portion is formed in such a manner as to act mainly on the injection object surrounded by the bag body. That is, when the first bag portion is expanded by being supplied with the expansion gas from the gas supply unit, an expanded portion thereof makes a pushing force act on the injection object rather than on the needleless syringe. For that reason, the injection object will be forced toward the needleless syringe as a result of the expansion of the first bag portion in the inside of the bag body. Therefore, the first bag portion can be arranged at least on the opposite side of the nozzle across the injection object and at a location in the vicinity thereof. Also, the first bag portion can be arranged at least at the nozzle side and in the vicinity thereof so as to fix the bag body itself with respect to the injection object. On the other hand, the second bag portion is formed in such a manner as to act mainly on the needleless syringe. That is, the second bag portion is arranged in the bag body in such a manner that the nozzle portion is sandwiched between the second bag portion and the injection object. As a consequence, when the second bag portion is expanded by the expansion gas, an expanded portion thereof will force the injection opening of the nozzle portion to the injection object side.

In this manner, in the bag body, the injection opening of the nozzle portion and the injection object will be made close with respect to each other in an effective manner by the above-mentioned actions assigned to the first bag portion and the second bag portion, respectively. For that reason, when the supply of the expansion gas from the gas supply unit to the individual bag portions is carried out, the injection support environment in which the injection object and the injection opening are in suitable contact with each other is formed, thus making it possible to achieve the injection of the injection objective substance by the needleless syringe in a suitable manner. Here, note that the supply of the expansion gas from the gas supply unit to the individual bag portions is not limited to a specific supply mode, as long as the contact between the injection object and the injection opening of the nozzle portion can be resultantly achieved in an appropriate manner. For example, the expansion gas may be supplied at the same time to both the bag portions from the gas supply unit, or the supply to either one of the bag portions may precede the supply to the other. In addition, the timing of supply and the amount of supply to each bag portion may be able to be arbitrarily adjusted.

Here, the above-mentioned needleless injection apparatus may be further provided with a pressure detection unit that is arranged at a location which is in the vicinity of said injection opening and at which a pressure applied to said injection opening from said injection object can be detected in a state where said expansion gas is supplied to said first bag portion and said second bag portion. By detecting the pressure applied to the injection opening by means of the pressure detection unit, a contact state of the injection opening with respect to the injection object can be estimated and grasped, thus making it possible to form a more suitable injection support environment. In addition, in the above-mentioned needleless injection apparatus, said pressure detection unit may be arranged between said injection object and said second bag portion in such a manner as to be pushed, upon expansion of said second bag portion, against said injection object together with said injection opening by the expansion of said second bag portion. By arranging the pressure detection unit in this manner, when the second bag portion is expanded by the expansion gas, the pressure detection unit will be placed under the same pressurized environment by expansion, as in the case of the injection opening. For that reason, the pressure detected by the pressure detection unit will reflect the contact state of the injection opening with respect to the injection object in a suitable manner, so formation of a more suitable injection support environment can be attained.

Moreover, the needleless injection apparatus described thus far may be further provided with a syringe control unit that is configured to control the injection of said injection objective substance in said needleless syringe. In that case, said syringe control unit is configured to carry out the injection of said injection objective substance, when said expansion gas is supplied from said gas supply unit and the pressure detected by said pressure detection unit reaches a predetermined pressure. With said syringe control unit constructed in this manner, there is formed an injection support environment in which the contact state of the injection opening and the injection object is made substantially constant. As a result of this, with respect to the contact state of the injection opening and the injection object, it becomes an adequate state, whereby an appropriate contact state for achieving suitable injection will be formed between the injection opening and the injection object, while suppressing an unpleasant feeling of the user due to excessive pressurization.

Here, the needleless injection apparatus described thus far may be further provided with a supply control unit that is configured to control the supply of said expansion gas in said gas supply unit. In that case, after said supply control unit supplies said expansion gas to said first bag portion and said second bag portion at predetermined timing, said syringe control unit carries out the injection of said injection objective substance. Thus, the supply control unit supplies the expansion gas at the predetermined timing which has been decided in advance, and by using it as a trigger, the syringe control unit automatically injects the injection objective substance, whereby the convenience of the user will be improved. Here, note that the injection of the injection objective substance being able to be automatically carried out by the needleless syringe in this manner is due to the fact that the suitable injection support environment is formed by the expansion of the first bag portion and the second bag portion, as mentioned above. In addition, said predetermined timing is a timing at which the injection objective substance should be injected, and which can be decided by a variety of means. For example, the predetermined timing may be decided by using a timer which is held by the supply control unit, etc., or the predetermined timing may be decided by using a detection result of a sensor or the like which detects a parameter relevant to the injection timing. For example, at a point in time when the pressure detection unit has sensed a predetermined pressure applied between the injection opening and the injection object (i.e., a state in which the nozzle is in abutment with the injection object at the predetermined pressure), the supply of the gas is once stopped by the supply control unit. Then, by using it as a trigger, the syringe control unit may control to inject the injection objective substance.

### ADVANTAGEOUS EFFECT OF THE INVENTION

It becomes possible to provide a needleless injection apparatus in which a suitable state of contact is formed between an injection opening from which an injection objective substance is injected and an injection object, in order to achieve suitable injection by a needleless syringe.

### BRIEF DESCRIPTION OF THE DRAWINGS

Fig. 1 is a cross sectional view of a needleless injection apparatus showing the schematic structure of the needleless injection apparatus according to the present invention.
Fig. 2 is a schematic view of the needleless injection apparatus showing a use or operation state thereof according to the present invention.
Fig. 3A is a view schematically showing a state before injection of the needleless syringe included in the needleless injection apparatus shown in Fig. 1.
Fig. 3B is a view schematically showing a state upon completion of injection of the needleless syringe included in the needleless injection apparatus shown in Fig. 1.
Fig. 4 is a view showing a way in which a nozzle member including nozzle flow passages is fitted to a nozzle holder, in the needleless syringe shown in Fig. 3A.
Fig. 5 is an enlarged view of the structure of the needleless syringe shown in Fig. 3B in the vicinity of the nozzle flow passages.
Fig. 6 is a flow chart of injection control carried out by the needleless injection apparatus shown in Fig. 1.
Fig. 7 is a second view showing the schematic structure of the needleless injection apparatus according to the present invention.
Fig. 8 is a third view showing the schematic structure of the needleless injection apparatus according to the present invention.

### EMBODIMENTS FOR CARRYING OUT THE INVENTION

Hereinafter, reference will be made to a needleless injection apparatus 40 according to embodiments of the present invention, while referring to the accompanying drawings. In addition, the needleless injection apparatus 40 is provided with a needleless syringe 20 (hereinafter, referred to simply as a "a syringe 20"). Here, note that the structure or construction of the following embodiments is only example, but the present invention is not limited to such structure or construction of the embodiments.

### First Embodiment

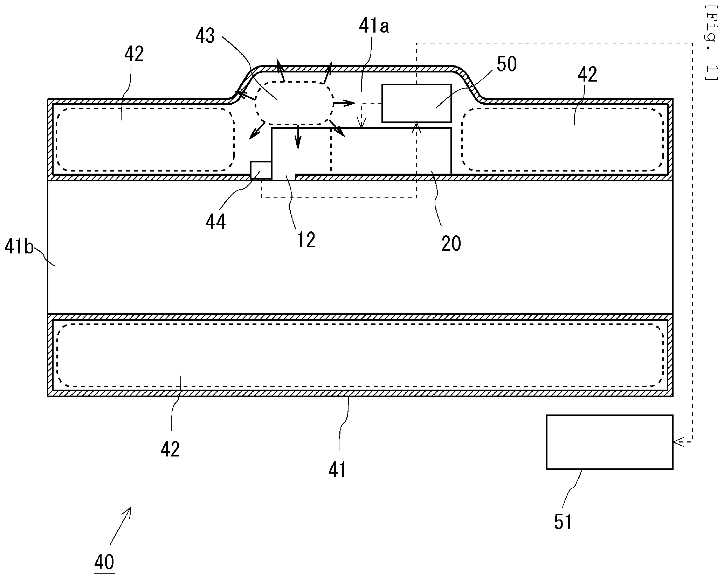

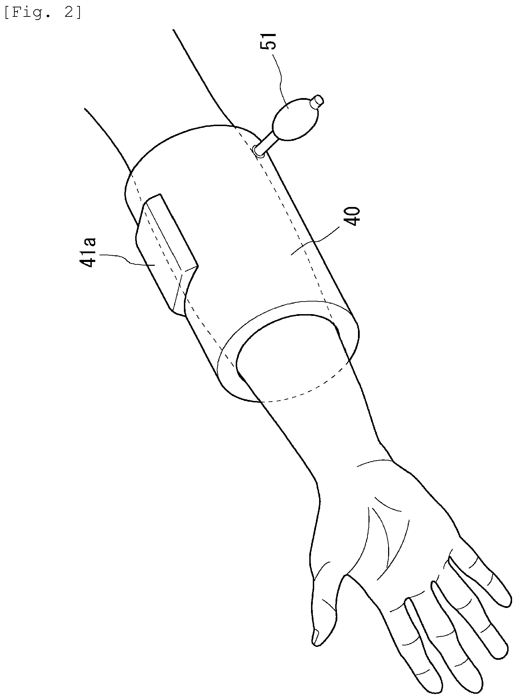

Here, Fig. 1 is a cross sectional view along an axial direction of the needleless injection apparatus 40, showing an internal state thereof, and Fig. 2 is a view showing a use or operation state of the needleless injection apparatus 40. In this embodiment, as shown in Fig. 2, a skin tissue of an arm, a foot or the like (hereinafter, generically referred to as an "arm", though it may be a foot) of a user becomes an injection object which is a target for injection of an injection solution to be injected by a syringe 20. Note that in the following description of this specification, the injection objective substance, which is to be injected into the injection object by the syringe 20, is generally referred to as "injection solution". However, this description includes no intention to limit the contents and the form of the substance to be injected. The component, which is to be delivered, for example, to a skin structure, may be either dissolved or not dissolved in the injection objective substance. Any specified form of the injection objective substance is available without any problem as well, for which various forms can be adopted, including, for example, liquid and gel form, provided that the injection objective substance can be discharged to the skin structure from an injection opening of each nozzle flow passage 3 to be described later by being pressurized.

The needleless injection apparatus 40 has a casing 41 of a cylindrical shape, and its surface is formed of a relatively easily deformable member such as cloth fiber, resin or the like, and so, when bag portions 42, 43 to be described later expand, the surface of the casing 41 is also caused to expand and deform in conformity with the expansion of the bag portions. In addition, a through space 41b extending through the casing 41 in the axial direction thereof is formed in the central portion of the casing 41. This through space 41b is to allow an arm of the user in the form of the injection object to pass therethrough, as shown in Fig. 2, and stated in another way, it is a space for mounting and positioning the needleless syringe 40 with respect to the user's arm. Accordingly, the size of the through space 41b is set to such a size as to enable the user's arm to be inserted therein.

Here, a space in the inside of the casing 41 other than the through space 41b is formed as a closed space which is closed by the member of the casing 41 (hereinafter, referred to simply as a "closed space"), and in the closed space, there are arranged a first bag portion 42, a second bag portion 43, the syringe 20, a pressure sensor (or pressure control unit) 44, and a control unit 50. The first bag portion 42 is a bag-shaped member which is expanded by air supplied from an air supply unit (or gas supply unit) 51 arranged in the outside of the casing 41, wherein the first bag portion 42 is arranged inside the casing 41 in such a manner as to substantially occupy, in the closed space, a space other than a central accommodation space 41a (refer to Fig. 2, too) which is a part of the central portion of the casing 41. That is, the first bag portion 42 is arranged in the closed space so as to annularly surround the user's arm (the injection object) inserted in the through space 41b except the central accommodation space 41a. Here, note that the air supply unit 51 is a unit which has a pump therein, and which supplies air for expansion to the first bag portion 42 and the second bag portion 43 to be described later, or discharges the supplied air from the individual bag portions.

Then, the syringe 20 is arranged at the side of the through space 41b in the central accommodation space 41a, wherein in such an arrangement state, the injection opening of the nozzle portion 12 for injecting the injection solution by means of the syringe 20 is exposed to the through space 41b. That is, although a large portion of the syringe 20 is accommodated in the central accommodation space 41a, the above-mentioned exposed state is formed in a state where the injection opening of the nozzle portion 12 is in opposition to the user's arm inserted in the through space 41b so as to be contactable therewith. Here, note that the detailed structure of the syringe 20 including the nozzle portion 12 will be described later. In addition, a pressure sensor 44 is arranged in the central accommodation space 41a in the vicinity of the injection opening of the nozzle portion 12, so as to perform the detection of pressure onto the arm, which is the injection object, generated in the injection opening.

Then, when the user's arm is inserted in the through space 41b, the second bag portion 43 is arranged at a location in the central accommodation space 41a at which the nozzle portion 12 is sandwiched between the second bag portion 43 and the arm in the through space 41b. This second bag portion 43 is a bag-shaped member which is expanded by the air supplied from the air supply unit 51, as in the case of the first bag portion 42. When the second bag portion 43 is expanded by this expansion air, it acts on the nozzle portion 12 so as to bring the injection opening thereof close to the user's arm in the through space 41b. Here, note that the syringe 20 is constructed such that it has a through hole 9 formed in its interior so as to enable a piston 7 to slide therein, as will be described later. For that reason, in Fig. 1, in order not to inhibit the sliding movement of this piston 7, the expansion of the second bag portion 43 serves to displace the entire syringe 20 by using a cavity and a deformable area inside the central accommodation space 41, thereby achieving that the injection opening of the nozzle portion 12 is brought close to the user's arm.

Further, the control unit 50 is also accommodated in the central accommodation space 41a. This control unit 50 has an arithmetic unit, a memory and so on incorporated therein, and achieves predetermined processing of the needleless injection apparatus 40 by executing a control program stored in the memory. In addition, the above-mentioned pressure sensor 44 is electrically connected to the control unit 50, and a pressure value measured by this sensor is handed over to the control unit 50, and is utilized for the predetermined processing, etc. Moreover, the syringe 20 and the air supply unit 51 are also electrically connected to the control unit 50, so that the injection of the injection solution in the syringe 20, and the supply and discharge of the expansion air by the air supply unit 51, are made controllable by means of the control unit 50. Accordingly, the control unit 50 corresponds to a syringe control unit and a supply control unit according to the present invention.

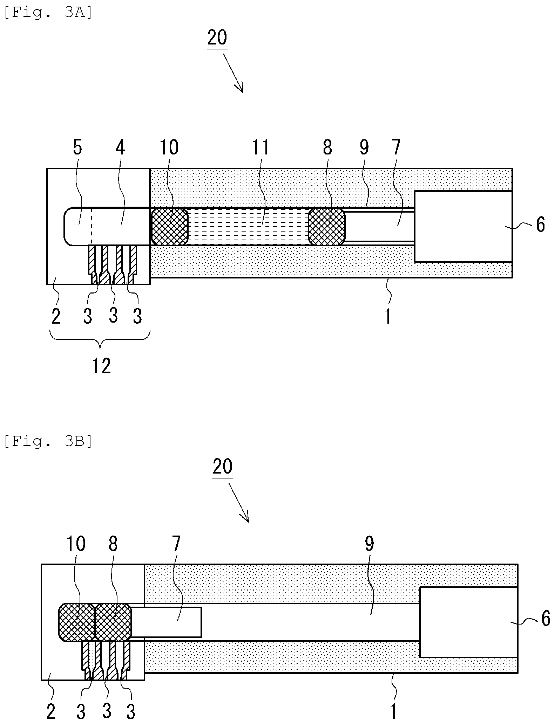

Here, the structure of the syringe 20 will be explained. Fig. 3A and Fig. 3B are cross sectional views along the axial direction of the syringe 20, wherein specifically, Fig. 3A is a view showing an internal state of the syringe 20 before injection, and Fig. 3B is a view showing an internal state of the syringe 20 upon completion of injection. Accordingly, in the state shown in Fig. 3A, the syringe 20 is in a state where the injection solution is filled therein, and on the other hand, in the state shown in Fig. 3B, the syringe 20 is in a state where the filled injection solution is injected to the injection object in the outside of the syringe 20.

In addition, the syringe 20 has a syringe main body 1, and in the central portion of the syringe main body 1, there is formed the through hole 9 which extends in the axial direction thereof, and which has a fixed diameter along the axial direction. Then, as will be described later, one end of the through hole 9 reaches the side of a pressure generation portion 6 (pressurization portion) which applies pressure to the injection solution filled in the syringe 20, and the other remaining end thereof reaches the side of a nozzle holder 2 (a holding member, hereinafter referred to simply as a "holder 2") in which the nozzle flow passages 3 are formed which serve as an injection path for the injection solution to the injection object. The nozzle portion 12 is formed by the holder 2 and the nozzle flow passages 3.

Here, the filled state of the injection solution in the through hole 9 will be explained. As shown in Fig. 3A, in the state before injection, the injection solution is filled in an accommodation space 11 which is formed inside the through hole 9 and which is sandwiched between an upstream side plug member 8 and a downstream side plug member 10. Here, note that the definition of "upstream" and "downstream" in this description is decided based on the flow of the injection solution which is generated by pressurization thereof at the time of injection. In addition, in order to transmit a driving force for injection of the injection solution generated in the pressure generation portion 6 to the injection solution filled in this manner, the piston 7 made of metal or resin is disposed in the through hole 9 at the upstream side of the upstream side plug member 8 (here, note that if the injection solution can be pressurized through the upstream side plug member 8, the piston 7 may not be necessarily provided). This piston 7 is disposed in the through hole 9 in such a manner that it has one end in contact with the upstream side plug member 8, and the other end able to receive the driving force (pressure) generated by the pressure generation portion 6. Thus, by way of the piston 7, the driving force from the pressure generation portion 6 can be transmitted to the injection solution, whereby the injection solution will flow toward the downstream side in the interior of the through hole 9, together with the upstream side plug member 8 and the downstream side plug member 10, and will be delivered to the side of the holder 2, as will be described later.

In order to achieve the above structure, materials for the upstream side plug member 8 and the downstream side plug member 10 are decided in such a manner that the injection solution may not leak out at the time of filling the injection solution, and that the injection solution can smoothly move in the interior of the through hole 9 accompanying the transmission of the driving force from the piston 7. For example, butyl rubber and silicone rubber can be adopted. Further, examples of the material include styrene-based elastomer, hydrogenated styrene-based elastomer, and the styrene-based elastomer and the hydrogenated styrene-based elastomer added with polyethylene, polypropylene, polybutene, polyolefin such as α-olefin copolymer, liquid paraffin, oil such as process oil, and powder inorganic matters such as talc, cast, and mica. Further, polyvinyl chloride-based elastomer, olefin-based elastomer, polyester-based elastomer, polyamide-based elastomer, and polyurethane-based elastomer, various rubber materials (in particular, those subjected to vulcanization) such as natural rubber, isoprene rubber, chloroprene rubber, nitrile-butadiene rubber, and styrene-butadiene rubber, mixtures of the kinds of elastomer and the kinds of rubber, and the like can be adopted.

Here, the pressure generation portion 6 will be explained. As one specific embodiment thereof, there can be adopted a pressure generation mode making use of combustion of propellant or pyrotechnic charge. For example, there can be utilized an initiator which combusts an ignition charge by electrical energization. The ignition charge is preferably exemplified by a propellant containing zirconium and potassium perchlorate (ZPP), a propellant containing titanium hydride and potassium perchlorate (THPP), a propellant containing titanium and potassium perchlorate (TiPP), a propellant containing aluminum and potassium perchlorate (APP), a propellant containing aluminum and bismuth oxide (ABO), a propellant containing aluminum and molybdenum oxide (AMO), a propellant containing aluminum and copper oxide (ACO), a propellant containing aluminum and iron oxide (AFO), or a propellant composed of a combination of a plurality of the foregoing propellants. These propellants exhibit such characteristics that, although the propellants generate hot and high-pressure plasma during combustion immediately after ignition, when combustion products condense at a room temperature, the propellants do not contain gaseous components and the pressure generated decreases abruptly. It is also allowable that any propellant or pyrotechnic charge other than the above is used as the ignition charge, provided that the injection can be performed appropriately.

Then, by transferring the inflammation or fire of the combustion products generated by the combustion of the ignition charge by means of the initiator to a gas generating agent which is separately arranged, the driving force to be transmitted from the pressure generation portion 6 to the piston 7 can be made more suitable. As for an example of the gas generating agent, it is possible to exemplify a single base smokeless propellant including 98% by mass of nitrocellulose, 0.8% by mass of diphenylamine, and 1.2% by mass of potassium sulfate. Further, it is also possible to use various gas generating agents used for a gas generator for airbags and a gas generator for seat belt pretensioners. With these gas generating agents, a predetermined gas generated at the time of combustion thereof contains a gas component even in room temperature, unlike the above-mentioned ignition charge, and hence, a relatively long combustion period of time can be ensured, thus making it possible to produce a sufficient driving force necessary for injection of the injection solution, as the pressure generation portion 6.

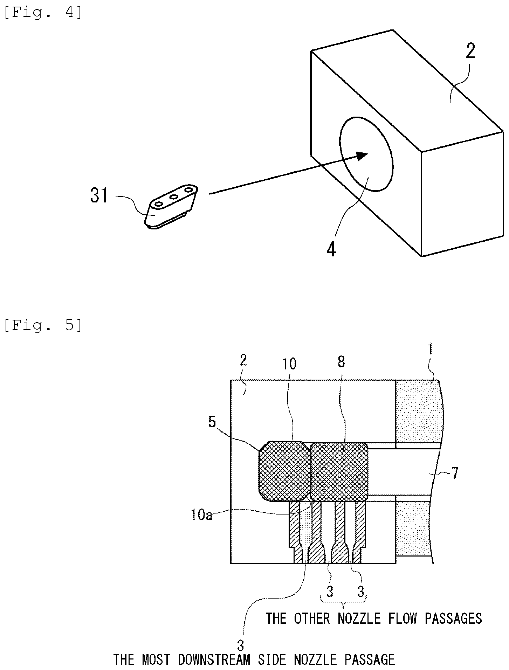

Here, at the tip end side of the syringe 20 (at the left side of Fig. 3A), the holder 2 including the nozzle flow passages 3 is arranged in the nozzle portion 12 for injecting the injection solution. For the nozzle flow passages 3, the nozzle portion 12 is formed by attaching to the holder 2 a nozzle attachment 31, which is constructed separately from the holder 2, as shown in Fig. 4, and the syringe itself can also be used for the next injection, by the nozzle attachment 31 being removed from the holder 2 after injection of the injection solution, and being exchanged for a new nozzle attachment 31. That is, in the syringe 20, in order to allow the nozzle once used to be replaced by a new one each time the injection of the injection solution is carried out, or in order to make the nozzle attachment 31 exchangeable according to the location or purpose of injection, it is constructed such that the syringe 20 is held to the holder 2 in a detachable manner. In addition, in order to enable such attachment and detachment (removal) of the nozzle attachment 31, it is constructed such that the holder 2 can be removed with respect to the syringe main body 1, and the details thereof will be described later.

Next, the detailed structure of the holder 2 will be explained. The holder 2 is mounted on the syringe main body 1, and in the holder 2, there is formed a concave portion 5 in which the downstream side plug member 10 moving with the injection solution under pressurization to the upstream side plug member 8 by the pressure generation portion 6 is finally accommodated. Specifically, the concave portion 5 is formed at the downstream side in the direction in which the downstream side plug member 10 is moved by the pressurization of the pressure generation portion 6 in the state where the holder 2 is mounted on the syringe main body 1, and the concave portion 5 has substantially the same diameter as the downstream side plug member 10, and has substantially the same depth as the length of the downstream side plug member 10. Then, there is formed a movement passage 4 between the initial position of the downstream side plug member 10 before the pressurization is carried out, i.e., the position of the downstream side plug member 10 shown in Fig. 3A, and the concave portion 5. As described above, the movement passage 4 and the concave portion 5 are arranged on the axis line of the through hole 9, thus making it possible for the downstream side plug member 10 to smoothly move and reach from the initial position to the concave portion 5 through the movement passage 4. In addition, the inside diameters of the through hole 9 and the movement passage 4 are formed to be substantially the same along the axial direction of the syringe 20, and the inside diameters of the through hole 9, the movement passage 4 and the concave portion 5 are formed to be substantially constant.

As constructed in this manner, when pressure is applied to the piston 7 by the pressure generation portion 6 so that the injection solution is moved to the tip end side of the syringe 20 together with the upstream side plug member 8 and the downstream side plug member 10, the downstream side plug member 10 is accommodated in the concave portion 5. When the downstream side plug member 10 is accommodated in the concave portion 5, the injection solution filled therein will be released, while also being accompanied by the pressurization from the upstream side plug member 8 to the injection solution, so that the injection solution will be injected from the injection openings of the nozzle flow passages 3 to the injection object outside the syringe through the movement passage 4.

Here, as shown in Fig. 5, in the holder 2, there are formed three (or one) flow passages 3 in a line along a pressurizing direction of the injection solution, in other words, along a direction of movement in which the injection solution is first moved by the pressurization of the pressure generation portion 6 and which is a direction toward the tip end of the syringe 20. Each of the nozzle flow passages 3 has its inlet directly open to the movement passage 4 and its outlet (or injection opening) open to the side of the syringe 20, but a nozzle flow passage 3 at the most downstream side (hereinafter, referred to the "most downstream side nozzle flow passage 3") is formed so as to have a function which is different from those of the other two nozzle flow passages 3 at the upstream side thereof (hereinafter, referred to as the "other nozzle flow passages 3").

A relative position of an opening portion of this most downstream side nozzle flow passage 3 with respect to the downstream side plug member 10 is decided in such a manner that the opening is not closed by the downstream side plug member 10 in a state where the downstream side plug member 10 is accommodated in the concave portion 5. In addition, a chamfer 10a is formed on the peripheral edge portion of a surface of the downstream side plug member 10 which is in opposition to the upstream side plug member 8. By ensuring passage of the injection solution into a substantially annular space formed by this chamfer 10a between the peripheral edge portion of the downstream side plug member 10 and the movement passage 4, a circulation state of the injection solution through the inlet of the most downstream side nozzle flow passage 3 is held, even in a state where the downstream side plug member 10 is accommodated in the concave portion 5. Moreover, when the downstream side plug member 10 is accommodated in the concave portion 5, the upstream side plug member 8 further pressurized comes closer to the downstream side plug member 10, so that it is finally placed in contact therewith. As a result, the injection solution filled between the downstream side plug member 10 and the upstream side plug member 8 flows into the three nozzle flow passages 3 opening there through the movement passage 4. Then, immediately before the upstream side plug member 8 contacts the downstream side plug member 10, the inlets of the other nozzle flow passages 3 excluding the most downstream side nozzle flow passage 3 will be closed by the upstream side plug member 8. However, even in the state where the upstream side plug member 8 is in contact with the downstream side plug member 10, by means of the above-mentioned chamfer 10a, the opening portion of the most downstream side nozzle flow passage 3 will not be in a state where it is closed by the downstream side plug member 10 and/or the upstream side plug member 8, but the inflow of the injection solution into the most downstream side nozzle flow passage through its inlet is held possible.

Thus, by the provision of the most downstream side nozzle flow passage 3 which has the inlet directly opening to the movement passage 4, and which is held in such a manner that the inflow of the injection solution from the movement passage 4 into this nozzle flow passage through the inlet thereof always becomes possible during the time after the downstream side plug member 10 is accommodated in the concave portion 5 until the upstream side plug member 8 contacts the downstream side plug member 10, it becomes possible to suppress or reduce the amount of the injection solution remaining in the syringe 20, in particular in the movement passage 4. In addition, the inlet of the most downstream side nozzle flow passage 3 opens directly to the movement passage 4, and the direction of injection of the injection solution defined by the inlet and the outlet (injection opening) (i.e., a direction from the interior of the syringe toward the side thereof, in the case of this embodiment) is nonparallel (in a direction in which the injection direction becomes orthogonal with respect to the direction of movement in Fig. 5) with respect to an initial direction of movement of the injection solution by pressurization (i.e., a direction toward the tip end of the syringe 20, in the case of this embodiment), and hence, in other words, the injection direction and the direction of movement are different from each other, so it is constructed such that the injection solution drawn into the nozzle flow passage from its inlet is injected to the outside through a relatively short path. For that reason, it is possible to exclude that a space (liquid reservoir space) in which the injection solution is apt to accumulate is formed in the interior of the syringe. As a result, efficient injection of the injection solution can be achieved. Moreover, with respect to the most downstream side nozzle flow passage 3, as shown in Fig. 5, at a point in time at which the injection is completed, the inflow of the injection solution into this nozzle flow passage 3 through its inlet is held, thus making it possible to suppress the formation of the above-mentioned liquid reservoir space and at the same time to reduce the residue of the injection solution in the movement passage 4 as much as possible.

In addition, among the three nozzle flow passages 3, also in the other nozzle flow passages 3 except the most downstream side nozzle flow passage 3, their inlets open directly to the movement passage 4, and hence, the injection of the injection solution to the injection object is achieved, while eliminating the formation of the liquid reservoir space, as described above. However, at the time of the completion of injection, the inlets of the other nozzle flow passages 3 will be directly closed by the upstream side plug member 8, or will be located at the upstream side of the upstream side plug member 8, as shown in Fig. 5, thus not contributing to the discharge of the injection solution until the last point in time of the completion of injection, unlike the most downstream side nozzle flow passage 3. However, by the provision of at least one most downstream side nozzle flow passage 3, as mentioned above, it is possible to suppress the remaining of the injection solution within the syringe 20. From this point of view, the syringe 20 may be constructed such that it is provided with a plurality of nozzle flow passages including the same discharge function as the most downstream side nozzle flow passage 3.

Moreover, in the above-mentioned embodiment, the inflow of the injection solution into the most downstream side nozzle flow passage 3 is held by the chamfer 10a of the downstream side plug member 10, but instead of this, a chamfer may be formed on the peripheral edge portion of a surface of the upstream side plug member 8 which is in opposition to the downstream side plug member 10, so that the inflow of the injection solution into the most downstream side nozzle flow passage 3 may be held by this chamfer, or a chamfer may be formed on the peripheral edge portion of the inlet of the most downstream side nozzle flow passage 3, so that the inflow of the injection solution into the most downstream side nozzle flow passage 3 may be held by this chamfer. Furthermore, the inflow of the injection solution into the most downstream side nozzle flow passage 3 may be held, by combining these chamfers in a suitable manner.

Here, note that in the above-mentioned embodiment, the three nozzle flow passages are formed in the holder 2 in a line along the direction of movement of the injection solution, but they are not limited to this and may be arranged in a plurality of lines. However, if the number of the nozzle flow passages increases, the residue (remaining amount) of the injection solution in the interior may increase, so it is preferable that the number of the nozzle flow passages be appropriate.

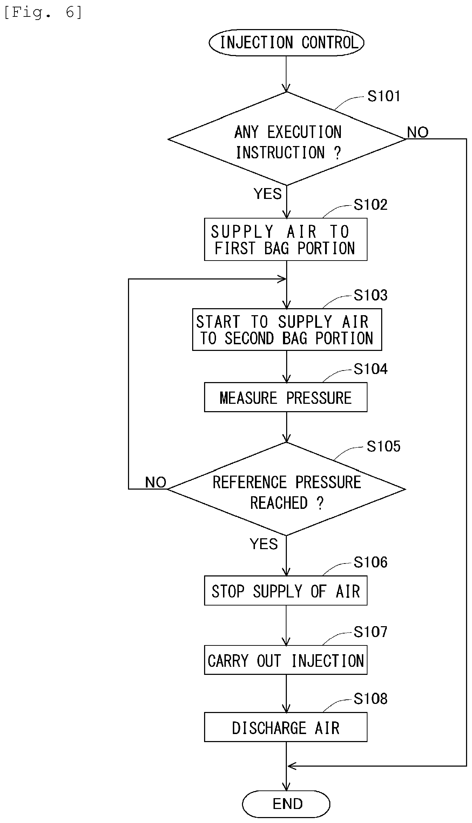

Here, the control with respect to the injection using the needleless syringe 20 in the needleless injection apparatus 40 will be explained based on Fig. 6. Fig. 6 is a flow chart of the injection control, wherein the injection control is carried out by the repeated execution of a predetermined control program at a predetermined interval in the control unit 50. First, in step S101, it is determined whether an instruction for carrying out injection using the needleless syringe 20 (hereinafter, referred to as an execution instruction) has been made. A variety of modes can be considered about determination of the presence or absence of the execution instruction. For example, when the current time point has reached a predetermined injection execution time which is set in an internal timer possessed by the control unit 50, a determination can be made that the execution instruction has been made. As another mode, when the user pushes an unillustrated depressible execution switch, or when an operation signal is supplied by remote control, etc., a determination can be made that the execution instruction has been made. This execution switch is arranged accessibly in an exposed state at a location easily accessible by the user, e.g., on an outer surface of the central accommodation space 41a, and is electrically connected to the control unit 50. Further, as yet another mode, when existence of an arm of the user is detected by an unillustrated sensor which detects that the user's arm, being the injection object, has been inserted into the through space 41b, a determination can be made that the execution instruction has been made. When an affirmative determination is made in step S101, the control flow or routine goes to step S102, whereas when a negative determination is made, this injection control is ended.

In step S102, according to an instruction from the control unit 50, a predetermined amount of air is supplied to the first bag portion 42 from the air supply unit 51. This predetermined amount of air is an amount of air which can apply such pressure as to lightly fix the arm in the through space 41b by the expansion of the first bag portion 42. Here, note that the volume occupied by the user's arm in the through space 41b differs from person to person, so the predetermined amount of air is to generate a certain pressure under which the user's arm does not move easily in the through space 41b, and at this point in time, the pressure applied to the syringe 20 is not adjusted strictly. After the processing of step S102 is terminated by completing the supply of the predetermined amount of air, the routine goes to step S103.

In step S103, in the state where the predetermined amount of air has been supplied to the first bag portion 42, the supply of air to the second bag portion 43 is started. This supply of air in step S103 is to gradually expand the second bag portion 43, and is carried out at such a speed or rate that the pressure applied to the injection opening of the nozzle portion 12 does not go up rapidly so as not to cause the user to feel displeasure or discomfort. Then, in step S104, the pressure applied to the injection opening of the nozzle portion 12 at that point in time is measured by the pressure sensor 44. The pressure sensor 44 is located at a side surface of the nozzle portion 12 (the holder 2), and is also located in the vicinity of the injection opening thereof, and hence, the pressure measured by the pressure sensor 44 can be rationally assumed as the pressure applied to the injection opening of the nozzle portion 12. After the processing of step S104 ends, the routine goes to step S105.

In step S105, it is determined whether the pressure measured in step S104 has reached a reference pressure. This reference pressure is a pressure in the case where a state of contact between the injection opening of the nozzle portion 12 and the surface (skin) of the arm, which is formed by the nozzle portion 12 being pushed out to the arm side, which is the injection object, by the expanded second bag portion 43, can be assumed as a suitable contact state for injection of the injection solution by the needleless syringe 20. That is, when the reference pressure is applied to the injection opening of the nozzle portion 12, it is assumed that the injection opening of the nozzle portion 12 is in contact with the surface of the arm with no gap, and that there is formed a contact state in which the user does not feel displeasure such as a pain, etc. When an affirmative determination is made in step S105, the routine goes to step S106, whereas when a negative determination is made, the processing in and after step S103 is carried out.

Subsequently, in step S106, when the measured pressure has reached the reference pressure, the supply of air to the second bag portion 43 is stopped. In this state, the arm in the through space 41b is subjected to pressure by means of the first bag portion 42 to which the predetermined amount of air is supplied, so that the arm is pushed up toward the injection opening of the nozzle portion 12 of the syringe 20. Then, by the air being supplied to the second bag portion 43, the nozzle portion 12 is pushed in toward the arm, and as a result, a state is formed where the injection opening of the nozzle portion 12 is in contact with the surface of the arm in a suitable manner. Accordingly, in the processing of step S107, the injection of the injection solution in the syringe 20 is carried out. Specifically, according to an instruction from the control unit 50, an ignition electric current is supplied to the ignition charge of the initiator in the pressure generation portion 6, whereby the pressurization of the injection solution and the injection thereof will be carried out.

Thereafter, in step S108, the air supplied to the first bag portion 42 and the second bag portion 43 is discharged. With this, the user's arm is released from the pressurization state caused by the expansion of the individual bag portions, so that it becomes possible to pull out the arm from the through space 41b.

Thus, according to this injection control, the injection of the injection solution by the syringe 20 is carried out, after the contact between the injection opening of the nozzle portion 12 and the surface of the user's arm is brought into a suitable state, by the action of the first bag portion 42 and the second bag portion 43. For that reason, the injection solution is injected under an injection support environment suitable for the syringe 20 including no injection needle, and it becomes easy to deliver the injection solution to a tissue which is at a desired position in the arm.

Here, note that in the above-mentioned injection control, the first bag portion 42 and the second bag portion 43 are made to expand sequentially in this order, but the first bag portion 42 and the second bag portion 43 may be made to expand at the same time, as long as the contact state of the nozzle portion 12 can be formed appropriately. In this case, in a situation where air is supplied to both the bag portions at the same time, the supply of the air to both the bag portions need only be stopped at the time when the measured pressure of the pressure sensor 44 reaches a reference pressure. Also, as another mode or method, the second bag portion 43 may first be made to expand, and the first bag portion 42 may then be made to expand. In this case, while expanding the first bag portion 42, the timing to stop the supply of air to the first bag portion 42 may be determined based on the measured value of the pressure sensor 44.

Further, the needleless injection apparatus 40 may also be provided with a plurality of syringes 20 in the casing 41. In that case, the user mounts on his or her arm the needleless injection apparatus 40 including the plurality of the syringes 20. Then, the injection execution timing for each of the syringes 20 is set in the control unit 50, whereby at the time of arrival of the injection execution timing for each syringe 20, the control unit 50 drives the air supply unit 51, and supplies air to the individual bag portions so that the contact between the injection opening of the nozzle portion 12 and the surface of the arm is brought into a suitable state, after which the injection of the injection solution in each syringe 20 is carried out in an automatic manner. In such a mode, only by mounting the needleless injection apparatus 40 on the arm, the user can be sequentially subjected to injections by the syringes 20 in a suitable manner.

### <First modified embodiment>

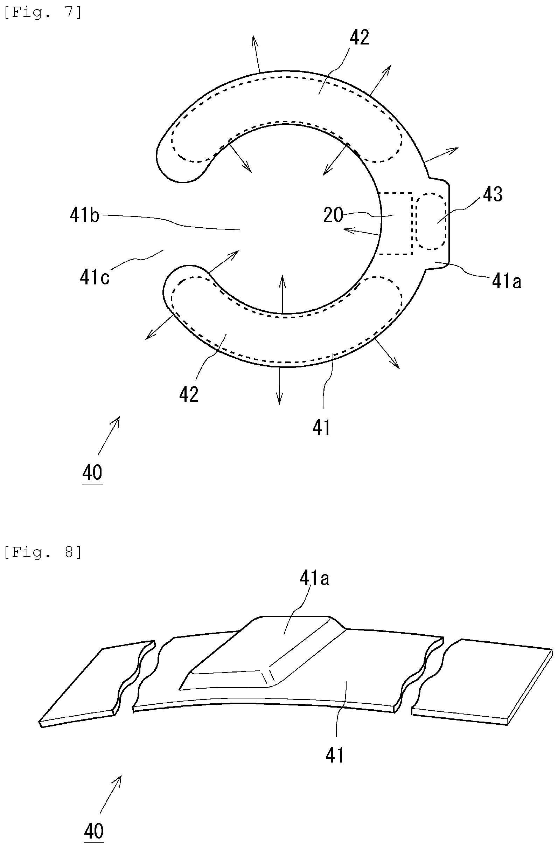

A modification of the needleless injection apparatus 40 will be explained based on Fig. 7. The needleless injection apparatus 40 according to this modification is constructed such that the casing 41 has a C shape in cross section (i.e., cross section vertical to an axial direction), and the other structure is the same as that of the needleless injection apparatus 40 of the above-mentioned embodiment. Here, note that an opening portion in the cross section is referred to by "41c" in Fig. 7. In the needleless injection apparatus 40 constructed in this manner, too, the injection opening of the nozzle portion of the syringe 20 can be made to contact the surface of the user's arm inserted in the through space 41b in a suitable manner, by the expansion of the first bag portion 42 arranged in the closed space inside the casing 41c, and the second bag portion 43 arranged in the central accommodation space 41a which is a part of the closed space. As a result, the injection of the injection solution by the syringe 20 including no injection needle is achieved in a suitable manner.

### <Second modified embodiment>

Another modification of the needleless injection apparatus 40 will be explained based on Fig. 8. In a state before the needleless injection apparatus 40 is mounted on the user's arm, the needleless injection apparatus 40 according to this modification has the casing 41 formed in a band shape, as shown in Fig. 8, and the other structure is the same as that of the needleless injection apparatus 40 of the above-mentioned embodiment. Then, when in use, by winding the band-shaped casing 41 around the user's arm and fixing it thereto, there is formed a mounting state generally similar to the state shown in Fig. 2. Accordingly, with the needleless injection apparatus according to this modification, too, the injection of the injection solution by the syringe 20 including no injection needle is achieved in a suitable manner.

### <Other embodiments>

According to the needleless injection apparatus 40 of the present invention, for example, cultured cells, stem cells, and the like may be seeded or inoculated into injection target cells or scaffold tissues (scaffolds) in the field of the regenerative medicine of human, in addition to the case where the injection liquid is injected into the skin structure. For example, as described in Japanese Patent Application Publication No. 2008-206477, the syringe 20 may inject cells which may be appropriately determined by those skilled in the art depending on a transplantation portion and the purpose of the cell regeneration, for example, endothelial cells, endothelial precursor cells, myeloid cells, preosteoblast, chondrocytes, fibroblast, skin cells, muscle cells, liver cells, kidney cells, intestinal tract cells, and stem cells, as well as all cells considered in the field of the regenerative medicine.

Further, the needleless injection apparatus 40 may be also used for delivering DNA or the like to cells or scaffold tissues (scaffolds) as described in Japanese Translation of PCT Application No. 2007-525192. In this case, it is possible to suppress an adverse effect on cells themselves or scaffold tissues (scaffolds) themselves when the needleless injection apparatus 40 is used, as compared with when the delivery is performed using a needle. Therefore, it can be said that the use of the needleless injection apparatus 40 is more desirable.

Further, the needleless injection apparatus 40 is ideally useful, for example, when various genes, cancer inhibiting cells, lipid envelops, and the like are directly delivered to target tissues and when antigen genes are administered to enhance the immunity against pathogens. In addition to the above, the needleless injection apparatus 40 can be also used, for example, in the field of medical treatment for various diseases (for example, see Japanese Translation of PCT Application No. 2008-508881 and Japanese Translation of PCT Application No. 2010-503616) and the field of immunological medical treatment (for example, see Japanese Translation of PCT Application No. 2005-523679). The field, in which the needleless injection apparatus 40 is usable, is not intentionally limited.

### DESCRIPTION OF THE REFERENCE SIGNS

1: syringe main body
2: holder (nozzle holder)
3: nozzle flow passages (most downstream side nozzle flow passage, the other nozzle flow passages)
6: pressure generation portion
7: piston
12: nozzle portion
20: syringe
40: needleless injection apparatus
41: casing
42: first bag portion
43: second bag portion
44: pressure sensor
50: control unit
51: air supply unit

## Claims

1. A needleless injection apparatus comprising:
an expansible bag body that is adapted to be arranged so as to surround an outer peripheral surface of an injection object;
a needleless syringe including no injection needle that comprises a nozzle portion for injecting an injection objective substance in said needleless syringe toward said injection object surrounded by said bag body, said nozzle portion including an injection opening which is in a state opposed to said injection object when said bag body is mounted on said injection object; and
a gas supply unit that supplies an expansion gas to said bag body;
wherein said bag body includes:
a first bag portion that is a bag portion adapted to expand by said expansion gas from said gas supply unit, said first bag portion being arranged at any of locations in said bag body except an arrangement location of said needleless syringe, in such a manner as to pressurize said injection object by expansion thereof; and
a second bag portion that is a bag portion adapted to expand by said expansion gas from said gas supply unit, and is formed separately from said first bag portion, said second bag portion being arranged at a location at which said nozzle portion is sandwiched between said second bag portion and said injection object, in such a manner as to push said injection opening toward said injection object by expansion thereof.

2. The needleless injection apparatus according to claim 1, further comprising:
a pressure detection unit that is arranged at a location which is in the vicinity of said injection opening and at which a pressure applied to said injection opening from said injection object can be detected in a state where said expansion gas is supplied to said first bag portion and said second bag portion.

3. The needleless injection apparatus according to claim 2, wherein
said pressure detection unit is arranged between said injection object and said second bag portion in such a manner as to be pushed, upon expansion of said second bag portion, against said injection object together with said injection opening by the expansion of said second bag portion.

4. The needleless injection apparatus according to claim 2 or 3, further comprising:
a syringe control unit that is configured to control injection of said injection objective substance in said needleless syringe;
wherein said syringe control unit is configured to carry out the injection of said injection objective substance when said expansion gas is supplied from said gas supply unit and the pressure detected by said pressure detection unit reaches a predetermined pressure.

5. The needleless injection apparatus according to claim 4, further comprising:
a supply control unit that is configured to control supply of said expansion gas in said gas supply unit;
wherein after said supply control unit supplies said expansion gas to said first bag portion and said second bag portion at predetermined timing, said syringe control unit carries out the injection of said injection objective substance.
